# EUROPEAN PATENT APPLICATION

(11) **EP 4 541 303 A1**
(43) Date of publication of application: **23.04.2025**
(21) Application number: 24206601.7
(22) Date of filing: 15.10.2024
(51) Int. Cl.: A61B 18/14, A61B 5/287

(54) **FLEXIBLE CIRCUIT ELECTRODES FOR LASSO CATHETER**

(30) Priority: 16.10.2023 US 202318487607
(71) Applicant: Biosense Webster (Israel) Ltd., 2066717 Yokneam (IL)
(72) Inventor: AGNEW V, William James, Irvine, 92618 (US)
(74) Representative: Carpmaels & Ransford LLP

(57) **Abstract**

An electrode assembly for a medical device can include a flexible substrate extending along a plane with a plurality of electrical leads extending along at least a portion of the flexible substrate. The electrode assembly can further include a plurality of electrodes disposed on the flexible substrate. Each electrode of the plurality of electrodes can be electrically connected to at least one electrical lead of the plurality of electrical leads. The plurality of electrodes can be positioned on the flexible substrate such, that when the electrode assembly is wrapped around an end effector member wrapped about a longitudinal axis to define a lasso catheter, at least some of the electrodes of the plurality of electrodes are positioned on a side of a lasso catheter facing away from the longitudinal axis extending through a lasso catheter.

## Description

### FIELD

The present invention relates generally to medical devices, and in particular, lasso catheters having improved flexibility and electrode arrangements.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. Electrical signals propagated through the heart can be mapped using a mapping catheter and then only selected areas of the tissue can be ablated to treat the AF. By selectively ablating cardiac tissue by application of energy via a catheter, it is sometimes possible to cease or modify the propagation of unwanted electrical signals from one portion of the heart to another. Medical probes may utilize radiofrequency (RF) electrical energy to heat tissue. Some ablation approaches use irreversible electroporation (IRE) to ablate cardiac tissue using nonthermal ablation methods.

Pulmonary vein isolation is a procedure commonly used to treat AF. Physicians typically use a lasso catheter comprising a plurality of ring electrodes clamped or otherwise attached to a spine of the lasso catheter. These ring electrodes, however, are not configured to measure electro-anatomical signals of tissue and electrical signals propagated through the surrounding blood simultaneously, which can reduce the effectiveness of a mapping operation and lead to an unnecessary increase in the total procedure time. Further, assembling ring electrodes on a lasso catheter can be a difficult and time-consuming process, thereby increasing the overall cost of the lasso catheter. Accordingly, there is a need in the art for devices configured to reduce the total procedure time by completing multiple elements of a mapping procedure with a single device capable of performing multiple functions simultaneously and applicable to a range of sizes. This and other issues can be addressed by the technology disclosed herein.

### SUMMARY

The disclosed technology includes an electrode assembly for a medical device. The electrode assembly can comprise a flexible substrate extending along a plane. The electrode assembly can further comprise a plurality of electrical leads extending along at least a portion of the flexible substrate. The electrode assembly can further include a plurality of electrodes disposed on the flexible substrate. Each electrode of the plurality of electrodes can be electrically connected to at least one electrical lead of the plurality of electrical leads. The plurality of electrodes can be positioned on the flexible substrate such, that when the electrode assembly is wrapped around an end effector member wrapped about a longitudinal axis to define a lasso catheter, at least some of the electrodes of the plurality of electrodes are positioned on a side of a lasso catheter facing away from the longitudinal axis extending through a lasso catheter.

The disclosed technology can include a medical probe comprising an insertion tube extending along a longitudinal axis. The medical probe can further comprise an end effector disposed at a distal end of the insertion tube. The end effector can be configured to bow radially outward from the longitudinal axis generally perpendicular to the longitudinal axis. The end effector can comprise a spine member and an electrode assembly disposed along at least a portion of the spine member. The electrode assembly can comprise a flexible substrate and a plurality of electrical leads extending along at least a portion of the flexible substrate. The electrode assembly can further comprise a plurality of electrodes disposed on the flexible substrate. Each electrode of the plurality of electrodes can be electrically connected to at least one electrical lead of the plurality of electrical leads. The plurality of electrodes can be wrapped around the spine member. At least some of the electrodes of the plurality of electrodes can be positioned facing away from the longitudinal axis.

The disclosed technology can further include a method for manufacturing a medical probe. The method can include forming a spine into a generally circular shape. The method can further include helically wrapping the spine with an electrode assembly. The electrode assembly can comprise a flexible substrate and a plurality of electrical leads extending along at least a portion of the flexible substrate. The electrode assembly can further comprise a plurality of electrodes disposed on the flexible substrate. Each electrode of the plurality of electrodes can be electrically connected to at least one electrical lead of the plurality of electrical leads. The plurality of electrodes can be positioned on the flexible substrate such that, when the electrode assembly is helically wrapped around the spine, at least some of the electrodes of the plurality of electrodes can be positioned to face away from an axis of the generally circular shape. These and other advantages of the disclosed technology will become more apparent throughout this disclosure in combination with the drawings.

### BRIEF DESCRIPTION OF THE DRAWINGS

FIG. 1 is a schematic pictorial illustration of a medical system including a medical probe having an end effector with electrodes, in accordance with an embodiment of the disclosed technology;
FIG. 2A is a bottom perspective view of an end effector for a medical probe, in accordance with the disclosed technology;
FIG. 2B is a top perspective view of an end effector for a medical probe, in accordance with the disclosed technology;
FIG. 2C is a side view of an end effector for a medical probe, in accordance with the disclosed technology;
FIG. 2D is a top view of an end effector for a medical probe, in accordance with the disclosed technology;
FIG. 3 is a side view of a spine member being wrapped in a flexible substrate, in accordance with the disclosed technology;
FIG. 4 is a cross-sectional view of a spine member of a medical probe taken along line X-X of FIG. 3, in accordance with the disclosed technology;
FIG. 5A is a top view of a flexible substrate having a plurality of electrodes, in accordance with the disclosed technology;
FIG. 5B is a side view of a spine member wrapped with the flexible substrate illustrated in FIG. 5A, in accordance with the disclosed technology;
FIG. 6A is a top view of an additional embodiment of a flexible substrate having a plurality of electrodes, in accordance with the disclosed technology;
FIG. 6B is a side view of a spine member wrapped in the flexible substrate illustrated in FIG. 6A, in accordance with the disclosed technology;
FIGS. 7A-B are detail views of a spine member wrapped in an insulated sleeve, in accordance with the disclosed technology; and
FIG. 8 is a flowchart illustrating a method of manufacture for a medical probe, in accordance with the disclosed technology.

### DETAILED DESCRIPTION

The disclosed technology includes electrodes disposed on a flexible substrate that can replace traditional ring electrodes employed in lasso catheters. The disclosed technology can help to ensure contact is maintained between at least one electrode disposed on a medical probe and target tissue during mapping of electro-anatomical signals at the pulmonary vein. Further, the disclosed technology can help ensure at least one electrode is not in contact with tissue but is in electrical communication with the surrounding blood to obtain reference signals that can then be used to eliminate noise that may be detected during the mapping procedure. As a result, the accuracy of a mapping procedure can be increased and the overall time required to complete a pulmonary vein isolation procedure can be reduced. Furthermore, the disclosed technology can help simplify the process of manufacturing the lasso catheter, thereby reducing the overall cost of the lasso catheter.

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives, and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 71% to 110%.

As used herein, the terms "patient," "host," "user," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example. As well, the term "proximal" indicates a location closer to the operator or physician whereas "distal" indicates a location further away to the operator or physician.

As discussed herein, "physician" or "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode including a high current density and high electric flux density is positioned at a treatment site, and a second electrode including comparatively lower current density and lower electric flux density is positioned remotely from the treatment site.

The disclosed technology can be configured to deliver monophasic or biphasic pulses to ablate tissue. For example, the electrodes described herein configured to deliver ablative energy to tissue can be configured to deliver monophasic pulses, biphasic pulses, or some combination thereof. The terms "biphasic pulse" and "monophasic pulse" refer to respective electrical signals. "Biphasic pulse" refers to an electrical signal including a positive-voltage phase pulse (referred to herein as "positive phase") and a negative-voltage phase pulse (referred to herein as "negative phase"). "Monophasic pulse" refers to an electrical signal including only a positive or only a negative phase.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation.

Example systems, methods, and devices of the present disclosure may be particularly suited for IRE ablation of cardiac tissue to treat cardiac arrhythmias. Ablative energies are typically provided to cardiac tissue by electrodes which can deliver ablative energy alongside the tissue to be ablated. Ablative procedures incorporating such example catheters can be visualized using fluoroscopy, magnetic-based position sensing, and/or active current location techniques.

IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by programmed cell death or apoptosis, which is believed to leave less scar tissue as compared to other ablation modalities. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

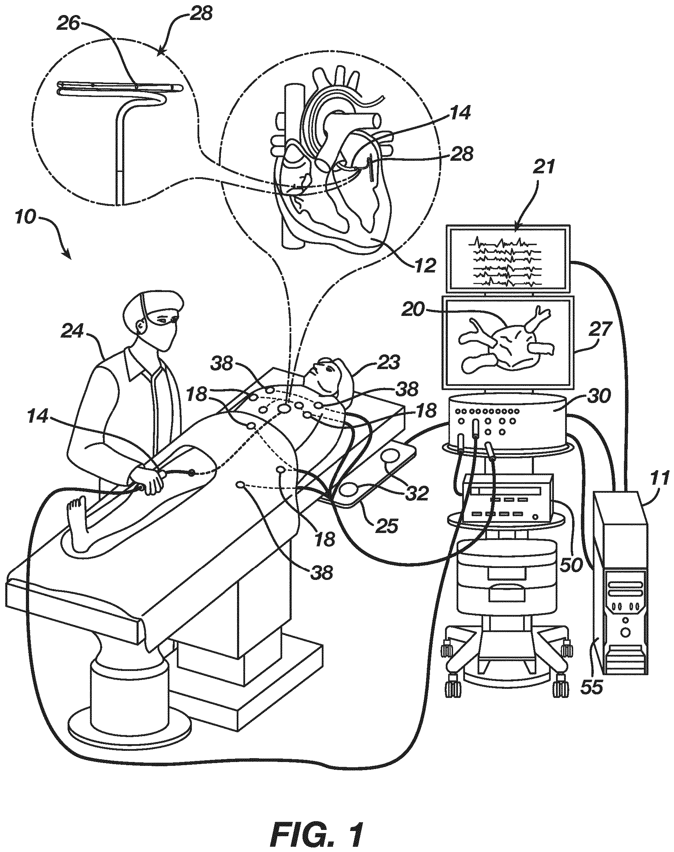

Reference is made to FIG. 1 showing an example catheter-based electrophysiology mapping and ablation system 10. System 10 includes one or more catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The one or more catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. For ablation, physician 24 brings end effector 28 comprising ablation electrodes to a target site for ablating. If the end effector 28 is alternatively or additionally configured for mapping of electrophysiological signals (e.g., IEGM signals), physician 24 similarly brings the end effector 28 into contact with the heart wall for sensing a target site in heart 12.

Catheter 14 is an exemplary catheter that includes an end effector 28 comprising one and preferably multiple electrodes 26 optionally distributed over an expandable assembly and a distal tip of end effector 28 and configured to detect electro-physiological signals and/or deliver ablative energy to tissue. Catheter 14 may additionally include a magnetic-based position sensor embedded in or near end effector 28 for tracking position and orientation of end effector 28. The end effector 28 can further include one or more impedance-based electrodes disposed in or near end effector 28 for tracking position and orientation of end effector 28.

Magnetic-based position sensor may be operated together with a location pad 25 including a plurality of magnetic coils 32 configured to generate magnetic fields in a predefined working volume. Real time position of end effector 28 of catheter 14 may be tracked based on magnetic fields generated with location pad 25 and sensed by magnetic-based position sensor 29. The magnetic-based position sensor can be a single axis sensor, a dual axis sensor, or a triple axis sensor depending on the particular configuration. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; and 6,892,091, each of which is incorporated herein by reference as if set forth fully herein.

System 10 includes one or more electrode patches 38 positioned for skin contact on patient 23 to establish location reference for location pad 25 as well as tracking of impedance-based electrodes. For impedance-based tracking, electrical current is directed toward impedance-based electrodes and sensed at electrode skin patches 38 so that the location of each electrode can be triangulated via the electrode patches 38. Details of the impedance-based location tracking technology are described in U.S. Patent Nos. 7,536,218; 7,756,576; 7,848,787; 7,869,865; and 8,456,182, each of which is incorporated herein by reference as if set forth fully herein.

A recorder 11 displays electrograms 21 captured with body surface ECG electrodes 18 and intracardiac electrograms (IEGM) captured with electrodes of catheter 14. Recorder 11 may include pacing capability for pacing the heart rhythm and/or may be electrically connected to a standalone pacer.

System 10 may include an ablation energy generator 50 that is adapted to conduct ablative energy to one or more electrodes disposed on the end effector and configured for delivering ablative energy to tissue. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618, USA.

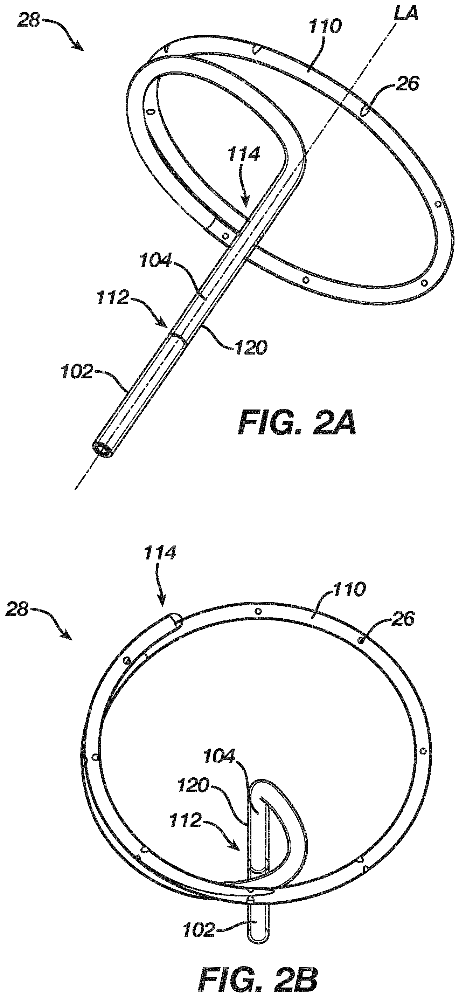

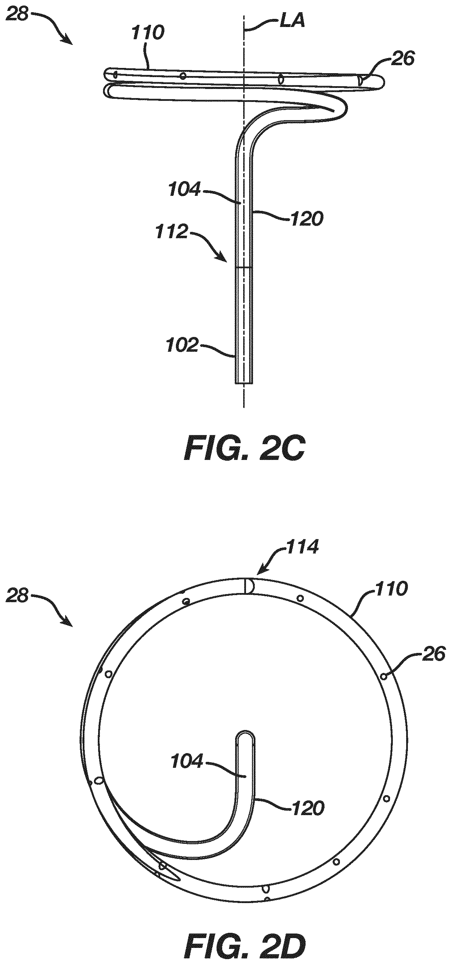

Turning now to FIGS. 2A-D, various views of the end effector 28 are shown and described. The end effector 28 can form a distal end of a medical probe. FIG. 2A is a top perspective view, FIG. 2B is a bottom perspective view, FIG. 2C is a side view, and FIG. 2D is a top view of the medical probe. As shown, the end effector 28 can be attached to a distal end of an insertion tube 102 extending along a longitudinal axis LA. The end effector 28 can be configured to bow radially outward from the longitudinal axis LA in a direction generally perpendicular to the longitudinal axis LA in a generally circular configuration. The end effector 28 can be attached to the insertion tube 102 at a proximal end 112, extend generally along the longitudinal axis LA a predetermined distance, and then bend approximately perpendicular to the longitudinal axis LA to form the circular configuration with a remainder of the end effector 28 to the distal end 114.

The end effector 28 can comprise a spine member 104 and an electrode assembly 150 disposed along at least a portion of the spine member 104. The spine member 104 can be made from a resilient material and be configured to form the generally circular shape of the end effector 28. The shape-memory material, for example, can be nitinol or other biocompatible materials that can be biased to form the generally circular shape when deployed from an insertion sheath. The electrode assembly 150 can include a flexible substrate 120 comprising a plurality of electrical leads 122 extending along at least a portion of the flexible substrate 120. The electrode assembly 150 can further include a plurality of electrodes 26 disposed on the flexible substrate 120 and each electrode 26 can be electrically connected to at least one electrical lead 122 of the plurality of electrical leads 122 and configured to detect electro-anatomical signals. The flexible substrate 120 can be wrapped around the spine member 104 of the end effector 28 and positioned such that at least some electrodes 26 of the plurality of electrodes are facing away from the longitudinal axis LA. In this way, the end effector 28 can comprise at least some electrodes 26 that are configured to contact tissue when inserted into a pulmonary vein.

Although the disclosed technology is described throughout this disclosure as being primarily for performing mapping of electro-anatomical signals, it will be appreciated that, alternatively or in addition, the disclosed technology can be configured for delivering ablative energy to tissue. For instance, the electrodes 26 described herein can be configured to detect electro-anatomical signals for a mapping procedure, for delivering ablative energy to tissue, or both. If the electrodes 26 are configured to deliver ablative energy to tissue, the electrodes 26 can be configured for RF or IRE ablation. Furthermore, the electrodes 26 can be configured to deliver unipolar or bipolar signals with monophasic or biphasic pulses.

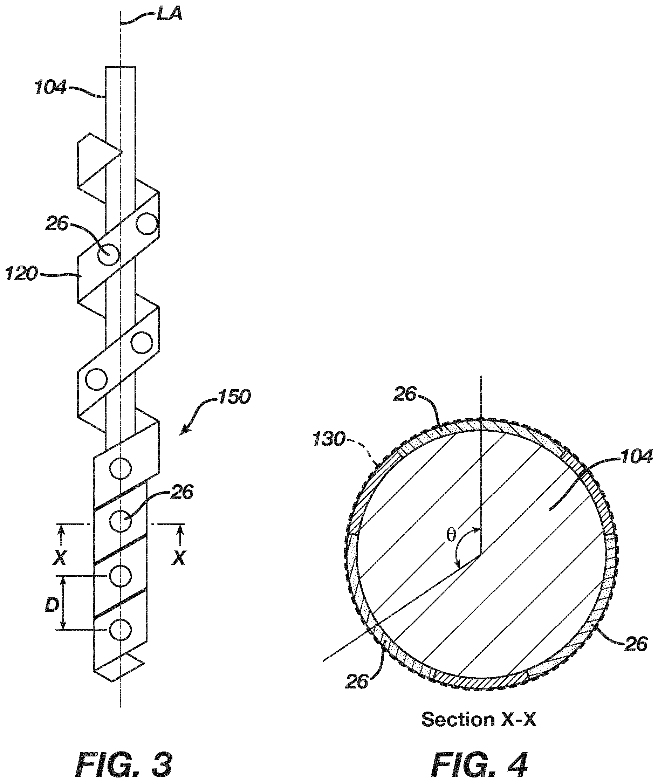

FIG. 3 illustrates a spine member 104 being wrapped in the flexible substrate 120. As previously described, the flexible substrate 120 can include a plurality of electrical leads 122 extending along at least a portion of the flexible substrate 120 and a plurality of electrodes 26 disposed on the flexible substrate 120. The flexible substrate 120, the electrical leads 122, and the electrodes 26 can define an electrode assembly 150. Each electrode 26 of the plurality of electrodes 26 can be electrically connected to at least one electrical lead 122 of the plurality of electrical leads 122. When the electrode assembly 150 is wrapped around the spine member 104, the plurality of electrodes 26 can be arranged along the flexible substrate such that each electrode 26 of the plurality of electrodes 26 is spaced a distance D from an adjacent electrode 26 of the plurality of electrodes 26. In some examples, the distance D can be between approximately 1 millimeter and 20 millimeters, between approximately 5 mm and 15 mm, or between approximately 8 millimeters and 12 millimeters. In other examples, the distance D can be approximately 9 millimeters.

The plurality of electrodes 26 can be arranged on the flexible substrate 120 such that multiple electrodes 26 are aligned around a circumference of the spine member 104. For example, at least two electrodes 26 can be aligned around a circumference of the spine member 104. In other examples, three, four, five, or more electrodes 26 can be aligned around a circumference of the spine member 104.

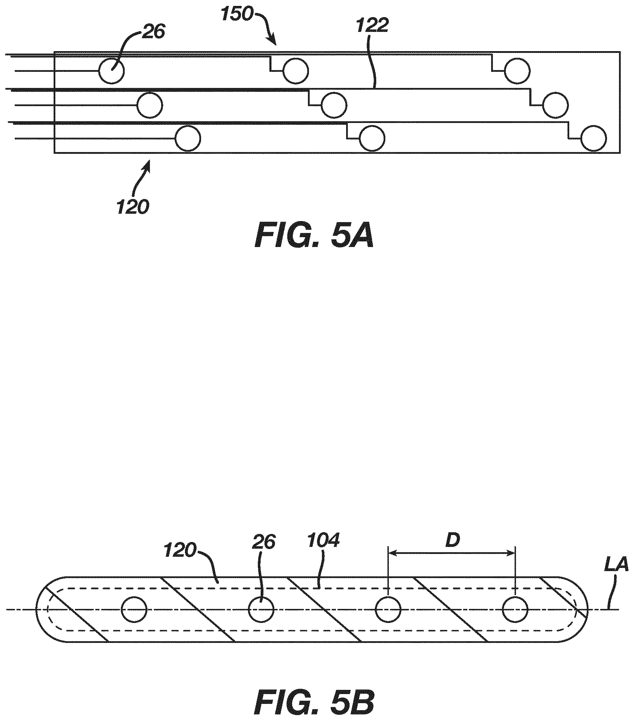

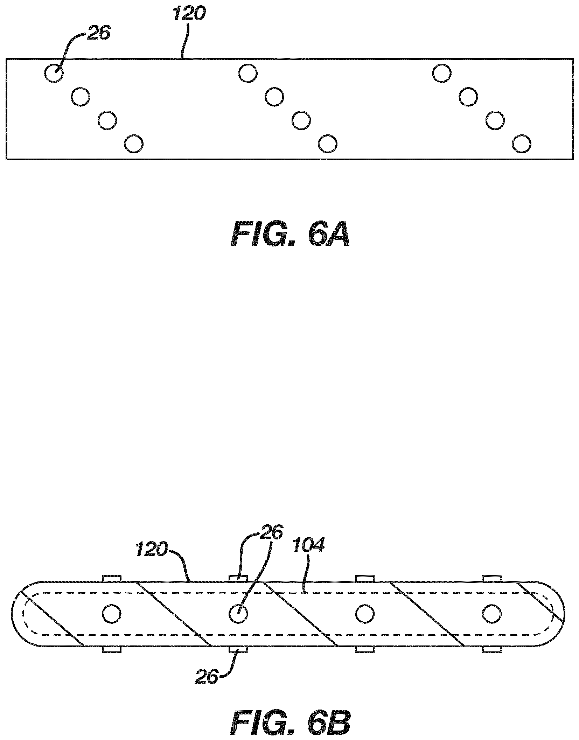

Additionally, as will be described in greater detail in relation to FIGs. 5A and 6A, it will be appreciated that the flexible substrate 120 can include groups of multiple electrodes 26 disposed on the flexible substrate 120 that are distributed along the flexible substrate 120 such that, when the flexible substrate 120 is wrapped around a spine member 104 of a medical probe, at least one electrode 26 is facing the tissue and at least one electrode 26 is facing the blood. Furthermore, an insulative sleeve 130 can be disposed over the electrode assembly 150 of the flexible substrate 120 once the flexible substrate 120 has been helically wrapped over the spine member 104.

FIG. 4 illustrates a cross-sectional view of the spine member 104 taken along line X-X of FIG. 3. As depicted and explained above, the flexible substrate 120 wrapped around spine member 104 can include a plurality of electrodes 26 arranged such that multiple electrodes 26 of the plurality of electrodes 26 can be generally aligned around a circumference of the spine member 104. Additionally, the electrodes 26 can be spaced from each other at an angle θ degrees apart from each other around the circumference of the spine member 104. In some examples, θ can be approximately 120 degrees. In another example, θ can be approximately 180 degrees, approximately 90 degrees, approximately 60 degrees, approximately 30 degrees, or other angles depending on the number of electrodes and the particular configuration. In some examples, the electrodes 26 can be equally spaced along the circumference of the spine member 106 or the electrodes can be unequally spaced (e.g., a greater number of electrodes 26 can configured to face generally away from the longitudinal axis or vice-versa). As will be appreciated, at least one electrode 26 of the multiple electrodes 26 can be configured to face tissue and another electrode 26 can be configured to face blood when the medical device is inserted into a lumen of a body.

Turning now to FIGs. 5A-5B, FIG. 5A illustrates a top view of an electrode assembly 150. The electrode assembly 150 can include a flexible substrate 120 and a plurality of electrical leads 122 disposed along a portion of the flexible substrate 120. The electrode assembly 150 can further include a plurality of electrodes 26 disposed on the flexible substrate 120, each electrode 26 of the plurality of electrodes 26 can be electrically connected to at least one electrical lead 122 of the plurality of electrical leads 122. FIG. 5B illustrates a side view of the flexible substrate 120 helically wrapped around a spine member 104. The flexible substrate 120 can be wrapped around the spine member 104 such that at least some electrodes 26 of the plurality of electrodes 26 are facing away from the longitudinal axis LA of the spine member 104. In FIGS. 5A-5B, the plurality of electrodes 26 can be arranged on the flexible substrate 120 such that at least three electrodes 26 are aligned around a circumference of the spine member 104 at each location where an electrode 26 is positioned facing away from the longitudinal axis LA. The electrodes 26 of the plurality of electrodes 26 can be spaced approximately 120 degrees apart around the circumference of the spine member 104, as depicted in FIG. 4. Additionally, the electrodes 26 can be spaced a distance D apart along the longitudinal axis LA of the spine member 104, as previously described. As will be appreciated, D can be a range of distances corresponding to the number of electrodes 26 that are disposed along the flexible substrate 120 and the particular configuration.

FIGS. 6A-6B illustrate a flexible substrate 120 that can include a plurality of electrodes 26 arranged on the flexible substrate 120 such that at least four electrodes 26 are aligned around a circumference of the spine member 104 at each location where an electrode 26 is positioned facing away from the longitudinal axis LA of the spine member 104. For example, the electrodes 26 can be configured to be spaced approximately 90 degrees from each other when the electrode assembly 150 is wrapped around the spine member 104. It will be appreciated that the plurality of electrodes 26 can include any number of electrodes 26 and that angle θ can be a range of degrees, granted that the number of electrodes 26 are spaced apart from each other around the circumference of the spine member 104. For example, two electrodes 26 of the plurality of electrodes 26 will be spaced approximately 180 degrees apart, five electrodes will be spaced approximately 72 degrees apart, etc. as will be understood by one generally skilled in the pertinent art.

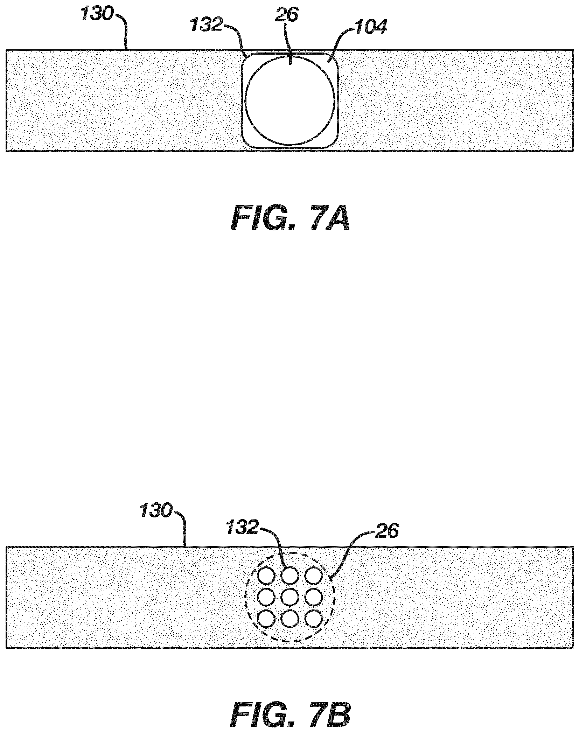

Turning now to FIGS. 7A-7B, FIG. 7A illustrates an insulative sleeve 130 that can be disposed over the electrode assembly 150 of the spine member 104, the electrode assembly 150 comprising a plurality of electrodes 26. The insulative sleeve 130 can comprise a plurality of apertures 132 extending through the insulative sleeve 130 such that each aperture 132 of the insulative sleeve 130 aligns with each electrode 26 of the plurality of electrodes 26. In other words, the apertures 132 can be configured to at least partially expose the electrode 26. In the example shown in FIG. 7A, the aperture 132 of the insulative sleeve 130 can partially expose a portion of the spine member 104.

The apertures 132 can be formed in the insulative sleeve 130 prior to installing the insulative sleeve 130 over the electrode assembly 150 and the spine member 104. Alternatively, the apertures 132 can be cut into the insulative sleeve 130 after installing the insulative sleeve 130 over the electrode assembly 150 and the spine member 104 to expose the electrodes 26.

FIG. 7B illustrates an insulative sleeve 130 that can be disposed over the electrode assembly 150 and the spine member 104. As before, the insulative sleeve 130 can comprise a plurality of apertures 132 extending through the insulative sleeve 130 to at least partially expose the electrodes 26. In this example, however, the apertures 132 can comprise multiple apertures 132 extending through the insulative sleeve 130 at each electrode 26 rather than one large aperture 132 as shown in FIG. 7A. In this way, the insulative sleeve 130 can be configured to provide mechanical protection to the electrode 26 in addition to providing an electrical insulation to the unexposed areas of the electrode assembly 150. The plurality of apertures 132 can be sized and spaced to ensure the electrode 26 is able to conduct sufficient electricity to or from the tissue and/or blood. In some examples, although not shown, the insulative sleeve 130 can comprise a plurality of irrigation holes extending through the insulative sleeve 130 that are each configured to permit an irrigation fluid to flow therethrough.

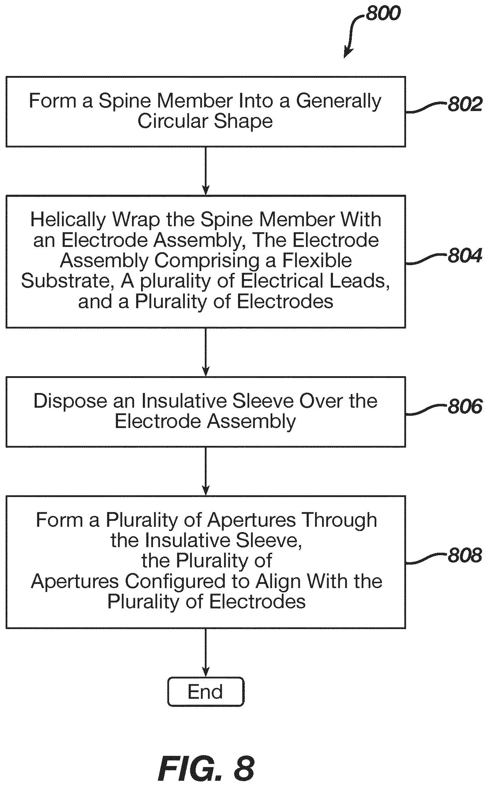

FIG. 8 illustrates a flowchart disclosing a method 800 of manufacturing a medical probe. Method 800 can include forming 802 a spine member 104 into a generally circular shape and helically wrapping 804 the spine member with an electrode assembly 150, the electrode assembly 150 comprising a flexible substrate 120, a plurality of electrical leads 122 extending along at least a portion of the flexible substrate 120, and a plurality of electrodes 26 disposed on the flexible substrate. The method 800 can include disposing 806 an insulative sleeve 130 over the electrode assembly 150 and forming 808 a plurality of apertures 132 through the insulative sleeve 130, the plurality of apertures configured to align with the plurality of electrodes 26.

In some examples, the plurality of electrical leads 122 of method 800 can be electrically connected to at least one electrical lead 122 of the plurality of electrical leads 122. Furthermore, the plurality of electrodes 26 can be positioned on the flexible substrate 120 such that, when the electrode assembly 150 is helically wrapped around the spine member 104, at least some of the electrodes 26 of the plurality of electrodes 26 can be positioned to face away from an axis of the generally circular shape of the spine member 104. As described above, the electrode assembly 150 can include any number of electrodes 26. In some examples, the medical probe can further include a position sensor (not shown) configured to generate a current when subjected to an electromagnetic field.

Regarding the examples above, in certain embodiments, the end effector 28 has a delivery configuration and a deployed configuration. In the delivery configuration, the end effector 28 is typically approximately straight, that is to say can be in a linear configuration to be progressed through and delivered to the heart through catheter 14. Once inside the heart and deployed from the catheter, the end effector 28 can take the lasso shape as illustrated herein. The positioning of the electrodes 26 both by distance D and angular displacement angle θ, is typically taken when the end effector is in the deployed configuration. Distance D and angle θ can differ when in the linear delivery configuration. Furthermore, as illustrated, the flexible substrate 120 can space the distance between electrodes 26 when linear to allow the distance D and angle θ to be met once wrapped around the spine 104 and deployed.

The disclosed technology described herein can be further understood according to the following clauses:
Clause 1: An electrode assembly for a medical device comprising: a flexible substrate extending along a plane; a plurality of electrical leads extending along at least a portion of the flexible substrate; and a plurality of electrodes disposed on the flexible substrate, each electrode of the plurality of electrodes electrically connected to at least one electrical lead of the plurality of electrical leads, the plurality of electrodes positioned on the flexible substrate such that when the electrode assembly is wrapped around an end effector member wrapped about a longitudinal axis to define a lasso catheter, at least some of electrodes of the plurality of electrodes are positioned on a side of a lasso catheter facing away from the longitudinal axis extending through a lasso catheter.
Clause 2: The electrode assembly of clause 1, each of the electrodes of the plurality of electrodes configured to detect electroanatomical signals.
Clause 3: The electrode assembly of clauses 1 and 2, the plurality of electrodes being helically arranged along the flexible substrate such that, each electrode of the plurality of electrodes is spaced approximately 9 millimeters from an adjacent electrode of the plurality of electrodes.
Clause 4: The electrode assembly of clause 1, the plurality of electrodes arranged on the flexible substrate such that at least three electrodes are generally aligned around a circumference of the lasso catheter at each location where an electrode is positioned to face away from the longitudinal axis.
Clause 5: The electrode assembly of clause 4, the at least three electrodes being spaced approximately 120 degrees apart from each other around the circumference of the lasso catheter.
Clause 6: The electrode assembly of clause 5, at least one electrode of the at least three electrodes being configured to face tissue and at least another electrode of the at least three electrodes being configured to face blood when the lasso catheter is inserted into a lumen of a body.
Clause 7: A medical probe comprising: an insertion tube extending along a longitudinal axis; an end effector disposed at a distal end of the insertion tube, the end effector configured to bow radially outward from the longitudinal axis generally perpendicular to the longitudinal axis, the end effector comprising: a spine member; and an electrode assembly disposed along at least a portion of the spine member, the electrode assembly comprising: a flexible substrate; a plurality of electrical leads extending along at least a portion of the flexible substrate; and a plurality of electrodes disposed on the flexible substrate, each electrode of the plurality of electrodes electrically connected to at least one electrical lead of the plurality of electrical leads, the plurality of electrodes wrapped around the spine member, at least some of the electrodes of the plurality of electrodes are positioned facing away from the longitudinal axis.
Clause 8: The medical probe of clause 7, each of the electrodes of the plurality of electrodes configured to detect electroanatomical signals.
Clause 9: The medical probe of clause 7, the plurality of electrodes arranged on the flexible substrate such that at least three electrodes are aligned around a circumference of the spine member at each location where an electrode is positioned facing away from the longitudinal axis.
Clause 10: The medical probe of clause 9, the at least three electrodes being spaced approximately 120 degrees apart from each other around the circumference of the spine member.
Clause 11: The medical probe of clauses 9 and 10, at least one electrode of the at least three electrodes being configured to face tissue and at least another electrode of the at least three electrodes being configured to face blood when the medical probe is inserted into a lumen of a body.
Clause 12: The medical probe of clause 7-11, the plurality of electrodes being arranged along the flexible substrate such that, when the electrode assembly is helically wrapped around the spine member, each electrode of the plurality of electrodes is spaced approximately 9 millimeters from an adjacent electrode of the plurality of electrodes.
Clause 13: The medical probe of clauses 7-12 further comprising an insulative sleeve disposed over the electrode assembly.
Clause 14: The medical probe of clause 13, the insulative sleeve comprising a plurality of apertures extending through the insulative sleeve, the plurality of apertures configured to align with the plurality of electrodes.
Clause 15: The medical probe of clause 13-14, the plurality of apertures comprising multiple apertures extending through the insulative sleeve at each electrode of the plurality of electrodes.
Clause 16: The medical probe of clause 13-15, the insulative sleeve comprising a plurality of irrigation holes extending through the insulative sleeve, the plurality of irrigation holes configured to permit an irrigation fluid to flow therethrough.
Clause 17: The medical probe of clauses 7-16, further comprising a position sensor configured to generate a current when subjected to an electromagnetic field.
Clause 18: A method comprising: forming a spine member into a generally circular shape; and helically wrapping the spine member with an electrode assembly, the electrode assembly comprising: a flexible substrate; a plurality of electrical leads extending along at least a portion of the flexible substrate; and a plurality of electrodes disposed on the flexible substrate, each electrode of the plurality of electrodes electrically connected to at least one electrical lead of the plurality of electrical leads, the plurality of electrodes positioned on the flexible substrate such that, when the electrode assembly is helically wrapped around the spine member, at least some of the electrodes of the plurality of electrodes are positioned to face away from an axis of the generally circular shape of the spine member.
Clause 19: The method of clause 18 further comprising disposing an insulative sleeve over the electrode assembly.
Clause 20: The method of clause 19 further comprising forming a plurality of apertures through the insulative sleeve, the plurality of apertures configured to align with the plurality of electrodes.

The embodiments described above are cited by way of example, and the present invention is not limited by what has been particularly shown and described hereinabove. Rather, the scope of the invention includes both combinations and sub combinations of the various features described and illustrated hereinabove, as well as variations and modifications thereof which would occur to persons skilled in the art upon reading the foregoing description and which are not disclosed in the prior art.

## Claims

1. An electrode assembly for a medical device comprising:
a flexible substrate extending along a plane;
a plurality of electrical leads extending along at least a portion of the flexible substrate; and
a plurality of electrodes disposed on the flexible substrate, each electrode of the plurality of electrodes electrically connected to at least one electrical lead of the plurality of electrical leads, the plurality of electrodes positioned on the flexible substrate such that when the electrode assembly is wrapped around an end effector member wrapped about a longitudinal axis to define a lasso catheter, at least some of electrodes of the plurality of electrodes are positioned on a side of a lasso catheter facing away from the longitudinal axis extending through a lasso catheter.

2. The electrode assembly of claim 1, each of the electrodes of the plurality of electrodes configured to detect electroanatomical signals.

3. The electrode assembly of claim 1 or claim 2, the plurality of electrodes being helically arranged along the flexible substrate such that each electrode of the plurality of electrodes is spaced approximately 9 millimeters from an adjacent electrode of the plurality of electrodes.

4. The electrode assembly of claim 1, the plurality of electrodes arranged on the flexible substrate such that at least three electrodes are generally aligned around a circumference of the lasso catheter at each location where an electrode is positioned to face away from the longitudinal axis, optionally the at least three electrodes being spaced approximately 120 degrees apart from each other around the circumference of the lasso catheter, further optionally at least one electrode of the at least three electrodes being configured to face tissue and at least another electrode of the at least three electrodes being configured to face blood when the lasso catheter is inserted into a lumen of a body.

5. A medical probe comprising:
an insertion tube extending along a longitudinal axis;
an end effector disposed at a distal end of the insertion tube, the end effector configured to bow radially outward from the longitudinal axis generally perpendicular to the longitudinal axis, the end effector comprising:
a spine member; and
an electrode assembly disposed along at least a portion of the spine member, the electrode assembly comprising:
a flexible substrate;
a plurality of electrical leads extending along at least a portion of the flexible substrate; and
a plurality of electrodes disposed on the flexible substrate, each electrode of the plurality of electrodes electrically connected to at least one electrical lead of the plurality of electrical leads, the plurality of electrodes wrapped around the spine member, at least some of the electrodes of the plurality of electrodes are positioned facing away from the longitudinal axis.

6. The medical probe of claim 5, each of the electrodes of the plurality of electrodes configured to detect electroanatomical signals.

7. The medical probe of claim 5, the plurality of electrodes arranged on the flexible substrate such that at least three electrodes are aligned around a circumference of the spine member at each location where an electrode is positioned facing away from the longitudinal axis, optionally the at least three electrodes being spaced approximately 120 degrees apart from each other around the circumference of the spine member, further optionally at least one electrode of the at least three electrodes being configured to face tissue and at least another electrode of the at least three electrodes being configured to face blood when the medical probe is inserted into a lumen of a body.

8. The medical probe of any of claims 5 to 7, the plurality of electrodes being arranged along the flexible substrate such that, when the electrode assembly is helically wrapped around the spine member, each electrode of the plurality of electrodes is spaced approximately 9 millimeters from an adjacent electrode of the plurality of electrodes.

9. The medical probe of any of claims 5 to 8 further comprising an insulative sleeve disposed over the electrode assembly.

10. The medical probe of claim 9, the insulative sleeve comprising a plurality of apertures extending through the insulative sleeve, the plurality of apertures configured to align with the plurality of electrodes.

11. The medical probe of claim 10, the plurality of apertures comprising multiple apertures extending through the insulative sleeve at each electrode of the plurality of electrodes.

12. The medical probe of any of claims 9 to 11, the insulative sleeve comprising a plurality of irrigation holes extending through the insulative sleeve, the plurality of irrigation holes configured to permit an irrigation fluid to flow therethrough.

13. The medical probe of any of claims 5 to 12, further comprising a position sensor configured to generate a current when subjected to an electromagnetic field.

14. A method comprising:
forming a spine member into a generally circular shape; and
helically wrapping the spine member with an electrode assembly, the electrode assembly comprising:
a flexible substrate;
a plurality of electrical leads extending along at least a portion of the flexible substrate; and
a plurality of electrodes disposed on the flexible substrate, each electrode of the plurality of electrodes electrically connected to at least one electrical lead of the plurality of electrical leads, the plurality of electrodes positioned on the flexible substrate such that, when the electrode assembly is helically wrapped around the spine member, at least some of the electrodes of the plurality of electrodes are positioned to face away from an axis of the generally circular shape of the spine member.

15. The method of claim 14 further comprising disposing an insulative sleeve over the electrode assembly, and optionally further comprising forming a plurality of apertures through the insulative sleeve, the plurality of apertures configured to align with the plurality of electrodes.
